# EUROPEAN PATENT APPLICATION

(11) **EP 1 840 203 A1**
(43) Date of publication of application: **03.10.2007**
(21) Application number: 06006835.0
(22) Date of filing: 31.03.2006
(51) Int. Cl.: C12M 1/40, C12P 7/64

(54) **Enzymatic modification in a continuously regenerated packed bed column**

(71) Applicant: Cargill, Inc., Wayzata, MN 55441-5624 (US)
(72) Inventor: Peeters, Esther Hendrika Gerarda, 4735 RG Zegge (NL); Kruidenberg, Marcus Bernardus, 3233 SL Oostvoorne (NL); Dell, Andrew James, Liverpool, L37 6DB (GB)
(74) Representative: Wibbelmann, Jobst

(57) **Abstract**

The invention relates to a process for the enzymatic modification of a substrate to produce a modified substrate which comprises the passing of the substrate through a packed bed column of a specific volume of immobilized enzyme with a flow of substrate in one direction and a periodic exchange of immobilized enzyme in the opposite direction. The substrate enters a packed bed column with a specific volume of immobilized enzyme at or near the bottom of the column and the modified substrate is discharged at or near the top of the column. A portion of the volume of the immobilized enzyme is periodically removed at the bottom of the column, while an equivalent portion of immobilized enzyme is periodically added at the top, thereby creating a flow of immobilized enzyme in the opposite direction of the substrate flow.

## Description

Enzymatic reactions are increasingly used for processing materials on an industrial scale. An important commercial step was the development of immobilized enzymes, enzymes that are physically attached to a solid support, either by adsorption or chemical bonds, to facilitate the separation of the enzyme from the reaction solution. Immobilization also enables multiple or repetitive use of the enzymes and often increases the stability of the enzymes.

Several types of reactors are used in conjunction with immobilized enzymes: packed bed reactors (also referred to as fixed bed reactors), fluidized bed reactors, stirred batch reactors, continuously stirred tank reactors, and membrane reactors. In large scale production, packed bed reactors are commonly used for particular applications. In such a reactor, the immobilized enzyme is packed in a column or as a flat bed while substrate and product streams are pumped into and out of the reactor, respectively. The main advantages of this type of reactor are the easy adaptation to larger scales, the high efficiency, low costs, the ease of operation and also an enhanced surface area per unit volume compared to membrane reactor systems (cf. W.M. Willis and A.G. Marangoni, Enzymatic Interesterification, in: Food Lipids - Chemistry, Nutrition, and Biotechnology, edited by C.C. Akoh and D.B. Min, pages 839-875).

A type of reaction in which the use of enzymes as catalysts became increasingly important in recent years in the oils and fats industry is interesterification. Interesterification is the exchange of acyl groups between an ester and an acid (acidolysis), an ester and an alcohol (alcoholysis) or between two esters (transesterification). The enzymes capable of interesterification reactions are lipases, classified as glycerol ester hydrolases (EC 3.1.1.3). For biotechnological purposes, these enzymes are predominantly obtained from bacterial, yeast, and fungal sources. These microorganisms secret lipases into their environment to digest lipid materials for subsequent uptake. In an aqueous environment, lipases catalyse the hydrolysis of triacylglycerides to produce diacylglycerides, monoacylglycerides, glycerol and free fatty acids. However, under water-limiting conditions, the reverse reaction, the synthesis of esters can also be achieved. Therefore, the direction of the reaction can be manipulated by regulating the water activity. At very low water concentrations ester synthesis predominates, above a water content of more than a few percent hydrolysis is the prevailing reaction, and in between, usually at a water content of less than 1% (w/v), transesterification is most effective.

Owusu-Ansar described in 1994 the use of immobilized lipases in a stirred tank reactor for the large-scale production of cocoa butter equivalents by lipase-catalysed interesterification (in: B.S. Carmail and Y. Kakuda (eds.) Technological Advances in Improved and Alternative Sources of Lipids, pages 360-389).

A packed bed interesterification process has been disclosed in WO 83/03844 wherein triacylglycerides are dissolved in an polar organic solvent. The lipase was immobilized by precipitation onto kieselguhr, hydroxyapatite or alumina particles.

WO 97/01632 is directed to another process for immobilization of an enzyme, specifically, a lipase or a phospholipase, for the processing of triglyceride oils.

Unilever further disclosed a two-stage process for production of cocoa butter equivalents and Betapol using packed bed columns (P. Quinlan & S. Moore, Inform 4, 580-585 (1993); A. Rozendaal & A.R. Macrae, in: F.D. Gansdone & F.B. Padlee (eds.) Lipid Technologies and Applications, 1997, pages 223-263).

X. Xu describes further processes with immobilized lipases, among them a packed bed reactor with a capacity of 10 kg/day (in U.T. Bornscheuer, Enzymes in Lipid Modiciation, 2000, pages 190-215).

The existing packed bed reactors suffer from the problem of a decrease in conversion rate over time. Though some enzymes such as lipases are quite stable, their efficacy inevitably drops over time, which results in lower reaction rates and non-uniform product quality. This lowering of quality can be counteracted by decreasing the flow rate and thereby increasing the residence time of the reactants in the reactor. Such a reduction in the flow rate, however, leads to a non-uniform output rate and while it can reduce the fluctuations in product quality, it cannot balance them completely. Furthermore, such a process often utilizes a number of discrete reactors in series which requires a complex flow control in order to run and maintain the system. The current invention solves these and related problems by using a new type of packed bed column process for enzymatic modification of a substrate.

### Summary of the invention

The invention relates to a process for the enzymatic modification of a substrate to produce a modified substrate which comprises the passing of the substrate through a packed bed column of a specific volume of immobilized enzyme with a flow of substrate in one direction and a periodic exchange of immobilized enzyme in the opposite direction. The substrate enters a packed bed column with a specific volume of immobilized enzyme at or near the bottom of the column and the modified substrate is discharged at or near the top of the column. A portion of the volume of the immobilized enzyme is periodically removed at the bottom of the column, while an equivalent portion of immobilized enzyme is periodically added at the top, thereby creating a flow of immobilized enzyme in the opposite direction of the substrate flow.

Whenever in this application reference is made to "near the bottom" or "near the top" the term "near" is intended to imply that material (e.g. substrate, modified substrate) is charged to or discharged from the reactor (through an opening, valve or similar) within a reasonable distance from said bottom or top determined by a skilled man. That could be 1 to 5%, or 10% of the length of a column measured from the top or bottom of the column referred to (for instance 5 cm, 10 cm, 15 cm etc., or 10 cm to 200 cm of a column having a length of 2 m).

### Detailed description of the invention

Hereinafter, the terms substrate and substrate solution are synonymously used to describe a liquid compound or mixture of compounds or a liquid solution of a compound or a mixture of compounds, which are meant to react in the presence of the immobilized enzyme. Thereby a liquid compound or mixture of compounds or a liquid solution of a compound or a mixture of compounds is produced, which is referred to as product or modified substrate, synonymously.

The invention relates to a process for the enzymatic modification of a substrate by passing the substrate through a packed bed column of immobilized enzyme. The substrate enters the packed bed column comprising a specific volume of immobilized enzyme at or near the bottom of the column and passes the column upward towards the top of the column, where the substrate exits the column at or near said top. Thus, the reactants enter the bed first at or close to the lowermost portion of said bed and leave the column at or close to its uppermost portion. In a preferred embodiment, the substrate and modified substrate streams are essentially continuous. By essentially continuous it is implied that the substrate and modified substrate streams can be stopped to allow for the periodic change of a portion of the volume of immobilized enzyme. However, there is no prolonged downtime necessary, e.g. for changing the whole volume of immobilized enzyme.

Periodically, a defined portion of the immobilized enzyme column is removed at the bottom of the column. This is done by the following procedure. The continuous flow of substrate is stopped. A discharge valve at the bottom of the column is opened and a defined portion of the slurry consisting of the immobilized enzyme material at the bottom end and substrate solution is released from the column. After a defined amount of this slurry was discharged, the discharge valve is closed and a feeding valve at the top or in a pipeline feeding towards the top of the reactor is opened, thereby allowing the entry of a fresh slurry of immobilized enzyme material and product at the top of the column. This fresh slurry will settle on the very top of the packed bed, the feeding valve is closed and the valves regulating the substrate and product streams are reopened.

By repeating this change of a portion of the bed material, a gradual flow of the immobilized enzyme from the top to the bottom of the column is assured. In consequence, the substrate solution will first come in contact with the most exhausted enzyme material and at the end with the freshest enzyme. This periodic change of a portion of the immobilized enzyme can be largely automated.

This system can be compared with multiple separate small basic reactors in series. In comparison to such a series, the complexity of the present invention is greatly reduced, particularly the number of input and output valves, and the processed solution has not to be transported between the multiple reactors. This is particularly important when the substrate and/or product solutions have to be kept at a certain temperature or when they have an increased viscosity. In addition, a system of small reactors requires that one or more of the reactors will periodically be out of service while spent enzyme is removed and fresh enzyme added. This is an intensive manual operation and during this time the efficiency of the entire system is greatly reduced. Accordingly, the overall capital and operating costs for such a system are not optimal.

In addition, the process according to the invention allows an essentially continuous flow rate as basically all substrate encounters the same amount of enzyme activity during its passage through the reactor. As the activity of the immobilized enzyme varies with age and use, the uniform contact of substrate with enzyme of various stages of activity is decisive to ensure a more constant degree of conversion. Therefore, a constant flow rate can be applied, while at the same time a more constant product quality is ensured.

The process of the present invention is particularly suited for an essentially continuous process. As the enzyme is periodically removed and replenished with relatively small quantities of fresh enzyme, the bed itself has a nearly constant quality. In addition, the reactor does not need a significant amount of downtime, e.g. for the change of bed material. This also reduces costs per amount of product and decreases the cost fluctuations. Another desirable aspect of this process is the comparably low operator involvement necessary. This not only reduces the associated costs, but also the probability of malfunction and thereby a further improvement of process stability can be achieved.

Periodically, utilized enzyme will be removed from the bottom of the column and fresh enzyme added to the top of the column. The frequency of this procedure will depend on the rate of activity decrease of the enzyme over time and the desired residence time of the substrate solution in the packed enzyme bed. The volume of enzyme material changed periodically and the length of the intervals between these changes can easily be adapted to the needs of the process and/or the substrate. In addition, there will be a relation between the frequency of such enzyme refreshing and the volume of enzyme that is refreshed. With all other process variables remaining constant, a small amount of enzyme can be refreshed frequently or a larger amount of enzyme refreshed less frequently. Preferably, a fixed percentage of the entire column volume (about between 0.3% and 3%) will be refreshed on a daily basis.

After passing through the packed bed column of immobilized enzyme, a sufficient conversion of the substrate should be reached. At the top of the column, product outlet is facilitated typically by small cylindrically shaped filter screens. These regulate the outflow of the product while the immobilized enzyme material is kept inside the reactor. There are two types of screens, vent screens and standard screens. While the standard screens are used for the actual outflow of product, the vent screens are higher up in the column and facilitate the flow of air or gas during draining and refilling.

The pumps regulating the input and output of the enzyme material slurries should be designed so that they do not break up the immobilized enzyme creating additional fines. This will assist an even flow and also to prevent fines from passing subsequent filtering devices.

The discharged enzyme will be a slurry of used immobilized enzyme and substrate solution. This slurry can be separated and the substrate solution can either be reintroduced into the tank which provides the input of substrate solution to the reaction column or blended with the product from the column. The used enzyme could be disposed of or further processed, depending on the remaining potential activity.

Optionally, the enzyme removed from the column can be subject to enzyme recycling for further use. For instance, in some cases the enzyme removed from the column still has some residual activity, which may be accessed if the enzyme material is broken into smaller particles, thereby revealing active sites of the enzyme that were not at all or just restrictedly accessible to the substrate. Alternatively, the enzyme is potentially able to be regenerated and reused. Therefore, in another embodiment of the process according to the invention, the enzyme material discharged from the column could be broken up or regenerated and reused in the process, e.g. pretreating the substrate prior to it entering the column.

### Example: Lipase Interesterification

To further demonstrate the design and operation of the process for enzymatic modification of substrates according to the invention, the use of this process for the interesterification of oils (triacylglycerides) with lipases is presented.

In the essentially continuous process described in this example, a substrate is pumped through one or more columns in parallel filled with immobilized enzyme. The flow of the oil mixture is opposite to the immobilized enzyme flow. The oil mixture input is at or near the bottom of the column and the fresh enzyme material is added at or near the top. Periodically, a part of the immobilized enzyme will be exchanged with fresh enzyme material. Therefore, the enzyme in the column has a nearly constant quality profile. The immobilized enzyme a the top of the column will be most fresh with nearly full activity and the immobilized enzyme near the bottom of the column will be nearly exhausted of activity. Accordingly, there will be a gradation of activity along the column length.

As used in the scope of the current invention the term "immobilized enzyme" is intended to mean any enzyme capable of performing an interesterification reaction which is affixed to a solid support of some kind. Solid support is typically any material either natural or synthetic that is designed to adsorb the prepared enzyme. Such solid supports are well know in the art and are commercially available from a variety of producers. Preferred supports such as silica are particularly useful in present invention. The enzyme, which is immobilized on the support, is preferably a lipase. Accordingly, an immobilized lipase enzyme is preferred in the present invention. The enzymes are available from a variety of sources and may be purchased on the market. Lypozyme TL IM (Novozymes) is a preferred enzyme in the present invention. The immobilized enzyme may be present in granules or agglomerated particles or any convenient form for the present invention.

As used in the scope of the current invention the term "substrate" is intended to mean a triacylglycerides, diacylglycerides, monoacylglycerides, or mixtures thereof. These acylglycerides may be of plant source including but not limited to palm kernel oil, palm oil, olive oil, rapeseed oil, canola oil, linseed oil, ground nut oil, soybean oil, cotton seed oil, sunflower seed oil, pumpkin seed oil, coconut oil, corn oil, castor oil, and walnut oil. In addition the substrate may be a single oil, a plant based oil fraction, such as a stearine, a single (mono, di, or tri) acylglyceride or mixtures of two or more of any of these possibilities. Stearines are well known in the art and are typically prepared by collecting the solid fraction from an oil after cooling to a point where some crystallization begins. The composition of the particular stearine will vary depending on the starting oil, the degree of cooling, and subsequent crystallization. The substrate may be utilized in a refined or unrefined state depending requirements. Preferred substrates for the purposes of the present invention include mixtures of palm stearine and coconut oil. These mixtures can range from 1-99 wt % palm stearine to 99-1 wt % coconut oil. Preferred mixtures include: 50% to 80 % by weight palm stearin and about 50% to 20% by weight coconut oil; and about 70% by weight palm stearin and about 30% by weight coconut oil.

For interesterification, it is usually preferable to guarantee a defined temperature of the process. As most of the interesterified oils and fats produced are intended for nutritional purposes, these products require the process to be free of organic solvents. Thus, the oils and fats are modified in a solvent-free system. Therefore, the process must be performed at temperatures which guarantee the substrates and products are in the liquid state. Lower temperatures could also increase the viscosity of the oil mixture, possibly culminating in partial solidification. This could create a non-ideal flow causing convection, back-mixing, channelling and/or the creation of stagnant regions within the column. Such variance could have a negative impact on the quality of the process and the interesterification product. Furthermore, the temperature should also be high enough to allow a high reaction rate which allows a reduction of the residence time necessary for the oil on the column to ensure sufficient conversion.

On the other hand, the temperature has to be low enough to not affect the stability and activity of the enzyme. Microbial lipases are quite thermostable and immobilization was found to further improve the stability of these enzymes so that the optimal temperatures for many lipases range from 30°C to 62°C (W.M. Willis and A.G. Marangoni, in Enzymatic Interesterification, Food Lipids - Chemistry, Nutrition, and Biotechnology, edited by C.C. Akoh and D.B. Min). Some lipases even allow temperatures of 60°C to 75°C (WO 97/01632). Future enzyme development could include even more temperature resistant varieties, e.g. resistant to 80°C, 90°C or above 100°C. At these temperatures, most triacylglycerides, even fully saturated ones, are liquefied and have a low enough viscosity to avoid non-ideal flow perturbances. Preferred temperatures for the present invention will be dictated by the enzyme utilized and the particular properties of the substrate including the minimum temperature required for the substrate to have a proper viscosity. For example the interesterification of palm stearine with coconut oil by Lypozyme TL IM (Novozymes) is preferably performed at about 70°C.

After heating the substrate oils to the desired temperature and mixing, the oil mixture is directed to the input valve at or near the bottom of the reactor filled with immobilized lipase. The reactor should be designed for vacuum and up to a suitable pressure. This pressure value is to allow for the maximum pressure drop over the enzyme bed in the column and the static pressure deriving from the column height. The pressure drop over the enzyme bed may depend on the characteristics of the enzyme particles, e.g particle size distribution, the flow rate of the substrate through the bed and the height of the bed. For instance, a suitable design pressure at a relatively high flow rate and relatively high enzyme bed might be less than 10 Bar. Another suitable design pressure at a relatively moderate too low flow rate and lower enzyme bed height might be for instance less than 6 Bar. To ensure that the feed pressure does not exceed this design pressure, the feeding pumps should not have a capacity above this design pressure. The inlets are further equipped with non-return valves, open/close valves, and flow rate control devices.

The fresh immobilized enzyme is supplied at the top of the column in regular intervals. A defined amount of interesterified product oil is pumped into a supply vessel equipped with a stirring device and immobilized enzyme added. Preferably, this supply vessel is situated above the reactor to facilitate enzyme loading. In a preferred embodiment, the vessel can further be flushed with nitrogen to prevent the oxidation of the oil. The fresh enzyme is added to the defined amount of product oil and mixed to give a free flowing slurry. To further enhance mixing and to improve the wetting of the pores of the immobilized enzyme, vacuum could be applied. When a fresh charge of enzyme is to be added, the supply vessel is aerated, if applicable, and the slurry or a portion thereof emptied into the reactor. If required, the vessel and pipelines can be flushed with a certain volume of product oil to ensure complete emptying of the vessel. The present invention is essentially continuous. In that it the inventors mean that the process may be run essentially around the clock with the exception of the few moments that it takes to exchange a portion of the enzyme from the column. This down time is only a very small fraction of the overall running time of the process.

## Claims

1. A process for the enzymatic modification of a substrate to produce a modified substrate comprising passing the substrate through a packed bed column of a specific volume of immobilized enzyme wherein:
a) the substrate enters the column at or near the bottom and the modified substrate exits at or near the top of the column,
b) a portion of the volume of immobilized enzyme is periodically removed from the bottom of the column, and
c) an equivalent portion of immobilized enzyme is periodically added to the top of the column.

2. A process according to claim 1 wherein the process is essentially a continuous process.

3. A process according to claim 1 or 2 wherein the removed portion of the volume of immobilized enzyme is used to pre-treat the substrate prior to the substrate entering the column.

4. A process according to claim 3 wherein the removed portion of the volume of immobilized enzyme is regenerated prior to it being utilized.

5. A process according to any one of claims 1 to 4 wherein the enzymatic modification is an interesterification, the enzyme is a lipase, and the substrate comprises one or more plant based oils or plant based oil fractions.

6. A process according to claim 5 wherein the substrate comprises a mixture of two or more plant based oils or plant based oil fractions.

7. A process according to any one of claims 5 to 6 wherein the substrate comprises triacylglycerides, diacylglycerides, monoacylglycerides, phospholipids, or free fatty acids.

8. A process according to claim 7 wherein the substrate further comprises methanol, ethanol, glycerol, monopropylene glycol, erythritol, pentaerythritol, sorbitol.

9. A process according to claim 6 wherein the substrate comprises about 50% to 80 % by weight palm stearin and about 50% to 20% by weight coconut oil.

10. A process according to claim 6 wherein the substrate comprises about 70% by weight palm stearin and about 30% by weight coconut oil.

11. A process according to any one of claims 5 to 10 wherein the substrate is refined prior to utilization.

12. A process according to any one of claims 5 to 11 wherein the process temperature is constantly kept between about 50°C and 80°C.

13. A process according to any one of claims 5 to 12 wherein the substrate is essentially free of organic solvents.
